(19) Europäisches Patentamt
European Patent Office
Office européen des brevets

(11) Veröffentlichungsnummer: **0 021 092**
**A1**

(12) # EUROPÄISCHE PATENTANMELDUNG

(21) Anmeldenummer: **80102981.0**

(51) Int. Cl.³: **C 07 C 69/74**

(22) Anmeldetag: **29.05.80**

(30) Priorität: **12.06.79 DE 2923774**

(43) Veröffentlichungstag der Anmeldung: **07.01.81**
**Patentblatt 81/1**

(84) Benannte Vertragsstaaten: **BE CH DE FR GB IT LI NL**

(71) Anmelder: **BAYER AG, Zentralbereich Patente, Marken und Lizenzen, D-5090 Leverkusen 1, Bayerwerk (DE)**

(72) Erfinder: **Maurer, Fritz, Dr., Roeberstrasse 8, D-5600 Wuppertal 1 (DE)**
Erfinder: **Priesnitz, Uwe, Dr., Heinrich-Heine-Strasse 42, D-4750 Unna-Massen (DE)**
Erfinder: **Riebel, Hans-Jochem, Dr., In der Beek 92, D-5600 Wuppertal 1 (DE)**

(54) **Verfahren zur Herstellung von 3,3-Dimethyl-cyclopropan-1,2-dicarbonsäureestern.**

(57) Die Erfindung betrifft ein neues Verfahren zur Herstellung von bekannten 3,3-Dimethyl-cyclopropan-1,2-dicarbonsäureestern der Formel I

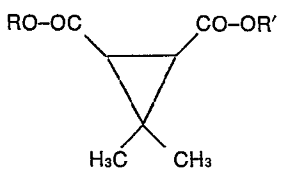

(I)

in welcher
R′ und R die in der Beschreibung angegebene Bedeutung haben, das dadurch gekennzeichnet ist, daß man 1,3-Dibrom-2,2-dimethyl-propan-1,3-dicarbonsäureester der Formel II

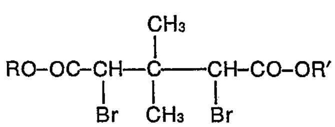

(II)

mit Zink, gegebenenfalls in Gegenwart eines Verdünnungsmittels, bei Temperaturen zwischen 20 und 200° C umsetzt.

EP 0 021 092 A1

0021092

BAYER AKTIENGESELLSCHAFT
Zentralbereich
Patente, Marken und Lizenzen

5090 Leverkusen, Bayerwerk
Rt/Kü
Typ IVa/ZP

Verfahren zur Herstellung von 3,3-Dimethyl-cyclopropan-1,2-dicarbonsäureestern

Die Erfindung betrifft ein neues Verfahren zur Herstellung von bekannten 3,3-Dimethyl-cyclopropan-1,2-dicarbonsäureestern, welche als Zwischenprodukte zur Herstellung von pestiziden Wirkstoffen verwendet werden können.

Es ist bereits bekannt geworden, daß man 3,3-Dimethyl-cyclopropan-1,2-dicarbonsäure-dimethylester erhält, wenn man Fumarsäure-dimethylester mit Dimethyl-diazomethan bei Temperaturen zwischen 0 und 5°C umsetzt und das dabei entstehende 1:1-Addukt in Gegenwart von Kupferpulver auf 16o bis 17o°C erhitzt (vergleiche J.Am.Chem. Soc. 82 (196o), 5255).

Diese Synthesemethode ist mit den durch die Verwendung von Dimethyl-diazomethan bedingten Sicherheitsrisiken behaftet; die Ausbeuten sind zudem unbefriedigend.

Weiter ist bekannt geworden, daß cis- bzw. trans-3,3-Dimethyl-cyclopropan-1,2-dicarbonsäure-dimethylester durch



BAD ORIGINAL

Umsetzung von Malein- bzw. Fumarsäure-dimethylester mit Diphenylsulfonium-isopropylid bei -7o°C in guten Ausbeuten synthetisiert werden können (vergleiche J.Am.Chem. Soc. 89 (1967), 3912-3914; ibid. 95 (1973), 397o-3976). Das bei dieser Synthesemethode als Ausgangsverbindung einzusetzende Diphenyl-sulfonium-isopropylid wird durch Umsetzung von Diphenyl-äthyl-sulfonium-tetrafluorborat mit Lithium-diisopropylamid, anschließende Alkylierung mit Methyliodid und erneute Umsetzung mit Lithium-diisopropylamid bei -7o°C hergestellt.

An Stelle von Diphenylsulfonium-isopropylid kann auch Triphenylphosphonium-isopropylid zur Synthese von 3,3-Dimethyl-cyclopropan-1,2-dicarbonsäureestern ausgehend von Malein- bzw. Fumarsäureestern verwendet werden (vergleiche Tetrahedron Lett. 1978, 1847-185o).

Die Umsetzungen mit den genannten Yliden werden wegen deren Empfindlichkeit gegen Luftsauerstoff und Feuchtigkeit unter einer Inertgasatmosphäre und unter Verwendung von sorgfältig getrockneten Lösungsmitteln durchgeführt. Das gleiche gilt auch für die Herstellung der Ylide; hierfür werden starke Basen,wie z.B. Butyllithium und/oder Lithium-diisopropylamid, benötigt.

Die oben erläuterten bekannten Synthesemethoden für 3,3-Dimethyl-cyclopropan-1,2-dicarbonsäureester sind wegen der aufwendigen Reaktionsführung, auch bei der Herstellung von Vorprodukten, für die Herstellung von 3,3-Dimethyl-cyclopropan-1,2-dicarbonsäureestern im industriellen Maßstab wenig geeignet.

Es wurde nun gefunden, daß man 3,3-Dimethyl-cyclopropan-1,2-dicarbonsäureester der Formel I

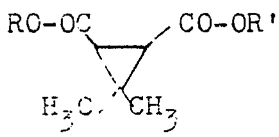

(I)

in welcher

R' und R gleich oder verschieden sind und für Alkyl stehen,

erhält,

wenn man 1,3-Dibrom-2,2-dimethyl-propan-1,3-dicarbonsäureester der Formel II

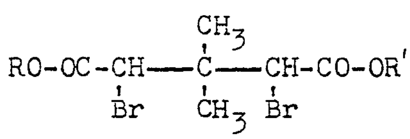

(II)

in welcher

R' und R gleich oder verschieden sind und für Alkyl stehen,

mit Zink, gegebenenfalls in Gegenwart eines Verdünnungsmittels, bei Temperaturen zwischen 2o und 2oo°C umsetzt.

Überraschenderweise können nach dem erfindungsgemäßen Verfahren 3,3-Dimethyl-cyclopropan-1,2-dicarbonsäureester der Formel (I) wesentlich einfacher und kostengünstiger als nach bekannten Methoden in sehr guten Ausbeuten und in hoher Reinheit hergestellt werden. Das neue Verfahren ist eher als die bisher bekannten



BAD ORIGINAL

Verfahren zur industriellen Herstellung von 3,3-Dimethyl-cyclopropan-1,2-dicarbonsäureestern geeignet.

Verwendet man als Ausgangsverbindung beispielsweise 1,3-Dibrom-2,2-dimethyl-propan-1,3-dicarbonsäure-dimethylester, so kann die Reaktion dieser Verbindung mit Zink durch folgendes Formelschema skizziert werden:

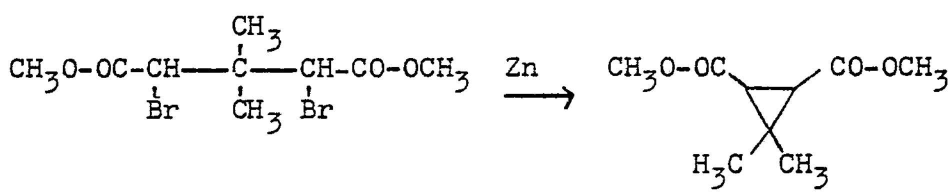


Die als Ausgangsverbindungen zu verwendenden 1,3-Dibrom-2,2-dimethyl-propan-1,3-dicarbonsäureester sind durch Formel (II) definiert. Vorzugsweise stehen darin R und R' für $C_1$-$C_4$-Alkyl, insbesondere für Methyl und Äthyl.

Als Beispiele seien genannt:
1,3-Dibrom-2,2-dimethyl-propan-1,3-dicarbonsäure-dimethylester, -diäthylester, -di-n-propylester, -di-iso-propylester, -di-n-butylester, -di-iso-butylester, -di-sek.-butylester und -di-tert.-butylester.

1,3-Dibrom-2,2-dimethyl-propan-1,3-dicarbonsäureester der Formel (II) sind bekannt (vergleiche J.Chem.Soc. (London) <u>79</u> (19o1), 754). Man erhält diese Verbindungen durch Umsetzung von 2,2-Dimethyl-propan-1,3-dicarbonsäure-derivaten mit Säurehalogeniden, wie z.B. Phosphor-pentabromid oder -pentachlorid, und Brom bei

Temperaturen zwischen 0 und 15o°C, und anschließende Umsetzung der Halogenierungsprodukte mit Alkoholen bei Temperaturen zwischen 1o und 5o°C. Zur Reinigung und Isolierung der Ester der Formel (II) wird mit Wasser verdünnt und mit einem mit Wasser nicht mischbaren Lösungsmittel, wie z.B. Äther oder Toluol, extrahiert. Die organischen Extrakte werden neutral gewaschen, getrocknet und eingeengt. Die im Rückstand verbleibenden Produkte können durch Vakuumdestillation gereinigt werden.

Das erfindungsgemäße Verfahren wird vorzugsweise unter Verwendung von Verdünnungsmitteln durchgeführt. Als solche kommen insbesondere aprotisch-polare Solventien in Betracht. Hierzu gehören insbesondere Carbonsäureamide wie z.B. Dimethylformamid, Dimethylacetamid und N-Methyl-pyrrolidin, Sulfoxide und Sulfone wie z.B. Dimethylsulfoxid und Tetramethylensulfon, Phosphorsäureamide wie z.B. Hexamethyl-phosphorsäuretriamid und Äther wie z.B. Glycol-dimethyläther, Diglycoldimethyläther, Tetrahydrofuran und Dioxan sowie Nitrile wie z.B. Acetonitril und Propionitril.

Die Reaktionstemperatur liegt beim erfindungsgemäßen Verfahren im allgemeinen zwischen 2o und 2oo°C, vorzugsweise zwischen 8o und 15o°C. Die Umsetzung wird im allgemeinen bei Normaldruck oder einem dem Dampfdruck des Verdünnungsmittels angepaßten Druck durchgeführt.

Zur Durchführung des erfindungsgemäßen Verfahrens setzt man auf 1 Mol 1,3-Dibrom-2,2-dimethyl-propan-1,3-dicarbonsäureester (II) zwischen 1 und 2,5 Mol, vorzugsweise zwischen 1,5 und 2,2 Mol Zink ein.

In einer bevorzugten Ausführungsform des erfindungsgemäßen Verfahrens wird das Zink in einem der oben angegebenen Verdünnungsmittel auf die erforderliche Reaktionstemperatur, vorzugsweise auf 9o bis 11o°C aufgeheizt und tropfenweise mit einem Ester der Formel (II) versetzt. Das Reaktionsgemisch wird nach Abklingen der exothermen Reaktion noch einige Stunden bei 1oo bis 12o°C gerührt, abgekühlt und abgesaugt. Zur Reinigung und Isolierung des Reaktionsproduktes wird das Filtrat mit einem mit Wasser nicht mischbaren Lösungsmittel, wie z.B. Toluol, versetzt, mit verdünnter Salzsäure und dann mit Wasser gewaschen, getrocknet und eingeengt. Das Reaktionsprodukt kann durch Vakuumdestillation gereinigt werden. Zur Charakterisierung dient der Siedepunkt.

Die nach dem erfindungsgemäßen Verfahren herzustellenden 3,3-Dimethyl-cyclopropan-1,2-dicarbonsäureester können als Zwischenprodukte zur Herstellung von insektizid und akarizid wirksamen Pyrethroiden verwendet werden (vergleiche Pestic. Sci. 7 (1976), 492-498; Tetrahedron Lett. 1978, 1847-185o).

Herstellungsbeispiel

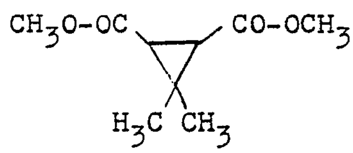

Zu einer Suspension von 9,8 g (o,15 g-Atom) Zinkstaub in 6o ml Dimethylformamid tropft man bei ca. loo°C 26 g (o,o75 Mol) 1,3-Dibrom-2,2-dimethyl-propan-1,3-dicarbonsäure-dimethylester. Bei der Zugabe steigt die Temperatur des Reaktionsgemisches auf ca. 13o°C an. Nach Ende der exothermen Reaktion hält man die Temperatur noch 2 Stunden bei 11o°C und kühlt dann auf Raumtemperatur ab. Das Gemisch wird mit 2oo ml Toluol versetzt und vom anorganischen Material abgesaugt. Das Filtrat schüttelt man einmal mit 5o ml lo%iger Salzsäure und dann zweimal mit je loo ml Wasser aus, trocknet es über Natriumsulfat und destilliert das Lösungsmittel im Vakuum ab. Durch Vakuumdestillation des Rückstandes erhält man lo,5 g (76% der Theorie) 3,3-Dimethylcyclopropan-1,2-dicarbonsäuredimethylester in Form einer farblosen Flüssigkeit mit dem Siedepunkt 94-97°C/7 mm Hg.

Patentanspruch:

1. Verfahren zur Herstellung von 3,3-Dimethyl-cyclopropan-1,2-dicarbonsäureester der Formel I

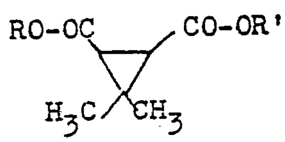

(I)

in welcher

R' und R gleich oder verschieden sind und für Alkyl stehen,

dadurch gekennzeichnet, daß man 1,3-Dibrom-2,2-dimethyl-propan-1,3-dicarbonsäureester der Formel II

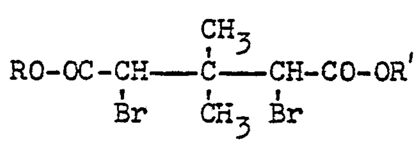

(II)

in welcher

R' und R gleich oder verschieden sind und für Alkyl stehen,

mit Zink, gegebenenfalls in Gegenwart eines Verdünnungsmittels, bei Temperaturen zwischen 20 und 200°C umsetzt.

0021092

Nummer der Anmeldung

Europäisches Patentamt

# EUROPÄISCHER RECHERCHENBERICHT

EP 80102981.0

| EINSCHLÄGIGE DOKUMENTE | | | KLASSIFIKATION DER ANMELDUNG (Int.Cl. 3) |
|---|---|---|---|
| Kategorie | Kennzeichnung des Dokuments mit Angabe, soweit erforderlich, der maßgeblichen Teile | betrifft Anspruch | |
| | DE - A - 1 921 842 (CIBA)<br>+ Patentansprüche 1,9-11 +<br>-- | 1 | C 07 C 69/74 |
| | DE - C - 965 580 (BAYER)<br>+ Patentanspruch +<br>-- | 1 | |
| | DE - A1 - 2 758 624 (RUSSEL-UCLAF)<br>+ Patentanspruch 1 +<br>-- | 1 | |
| | DE - A1 - 2 724 734 (AMERICAN CYANAMID)<br>+ Patentanspruch 1 +<br>-- | 1 | RECHERCHIERTE SACHGEBIETE (Int. Cl. 3)<br>C 07 C 69/00<br>C 07 C 19/00<br>C 07 C 121/00 |
| | DE - A1 - 2 615 160 (RUSSEL-UCLAF)<br>+ Patentanspruch 1 +<br>-- | 1 | |
| | DE - A - 1 668 613 (RHONE-POULENC)<br>+ Patentanspruch 1 +<br>---- | 1 | |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| WIEN | 27-08-1980 | REIF |